(19) Europäisches Patentamt
European Patent Office
Office européen des brevets

(11) EP 1 825 865 B1

(12) **EUROPÄISCHE PATENTSCHRIFT**

(45) Veröffentlichungstag und Bekanntmachung des Hinweises auf die Patenterteilung:
**14.01.2009 Patentblatt 2009/03**

(21) Anmeldenummer: **07002494.8**

(22) Anmeldetag: **06.02.2007**

(51) Int Cl.:
***A61K 47/10*** *(2006.01)* ***A61K 47/12*** *(2006.01)*
***A61K 47/18*** *(2006.01)* ***A61K 38/55*** *(2006.01)*
***A61K 47/34*** *(2006.01)* ***A61K 38/57*** *(2006.01)*

(54) **Stabilisierte Zubereitungen von Serin-Endopeptidasen, deren Herstellung und Verwendung**

Stabilised preparations of serine endopeptidases, their production and application

Préparations stabilisées de sérines endopeptidases, leur fabrication et utilisation

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **24.02.2006 DE 102006008613**

(43) Veröffentlichungstag der Anmeldung:
**29.08.2007 Patentblatt 2007/35**

(73) Patentinhaber: **Siemens Healthcare Diagnostics Products GmbH**
**35041 Marburg (DE)**

(72) Erfinder:
- **Lichte, Andrea. Dr**
  **35037 Marburg (DE)**
- **Kräling, Verena**
  **35099 Burgwald (DE)**

(56) Entgegenhaltungen:
**WO-A-20/05042012** **US-B2- 6 514 940**
**US-B2- 6 737 405**

- **DATABASE CA [Online] CHEMICAL ABSTRACTS SERVICE, COLUMBUS, OHIO, US; WU, VAN-YU ET AL: "Preservation of factor Xa activity" XP002457370 gefunden im STN Database accession no. 90:116915 & THROMBOSIS RESEARCH , 13(4), 701 CODEN: THBRAA; ISSN: 0049-3848, 1978,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung liegt auf dem Gebiet der Herstellung von Zubereitungen, die für eine therapeutische Verwendung oder für eine Verwendung als Testreagenz in einem diagnostischen Verfahren vorgesehen sind und betrifft insbesondere Zubereitungen von Serin-Endopeptidasen, die durch den Zusatz verschiedener Additive eine verbesserte Stabilität und damit Haltbarkeit aufweisen.

Proteasen (synonym: Peptidasen) sind Enzyme, die über die Fähigkeit verfügen, Peptidbindungen zu hydrolysieren. Die Stabilität von Zubereitungen, die Proteasen enthalten, ist einer der wichtigsten Parameter, die die kommerziellen Anwendungsmöglichkeiten von Proteasen in industriellen Prozessen mitbestimmen. Die Stabilität der Proteasen muss während ihrer Produktion, Isolierung, Reinigung, Lagerung und letztendlich auch bei der Anwendung des Produktes, das die Protease enthält, berücksichtigt werden. Damit eine ausreichende biologische Aktivität über einen akzeptablen Zeitraum gewährleistet werden kann, werden Zubereitungen, die eine Protease enthalten, wie viele andere Proteinprodukte auch, üblicherweise unter gekühlten Bedingungen oder sogar gefriergetrocknet gelagert.

Zur Stabilisierung von Enzymen oder Proteinen im Allgemeinen sind unterschiedliche Strategien bekannt. Das Ziel der Stabilisierungsstrategien ist im Wesentlichen die Vermeidung der Denaturierung des Proteins. Als native Struktur eines Proteins wird im Allgemeinen die Proteinkonformation bezeichnet, die die stabilste ist (Konformation mit der minimalsten Energie) und die das Protein in seiner zellulären Umgebung einnimmt oder bei der das isolierte Protein seine maximale biologische Aktivität besitzt. Die Denaturierung eines Proteins bezeichnet den Prozess, der zu einer Änderung der dreidimensionalen nativen Proteinstruktur führt, ohne dass die Aminosäuresequenz (Primärstruktur) verändert wird. Eine Änderung in der Molekülstruktur eines Enzyms kann Auswirkungen auf die korrekte Anordnung seines aktiven Zentrums haben und daher zu einer Inaktivierung des Enzyms führen. Unter der Stabilisierung eines Proteinmoleküls bzw. einer Zubereitung, die Proteinmoleküle enthält, versteht der Proteinchemiker die Vermeidung von Konformationsänderungen innerhalb des Proteinmoleküls. Stabilisierung führt folglich zur Erhaltung der nativen Struktur und damit auch zur Bewahrung der biologischen Aktivität. Über die Bestimmung der Aktivität eines Enzyms, beispielsweise einer Protease, können umgekehrt Rückschlüsse auf die Unversehrtheit der nativen Proteinstruktur gezogen werden.

Im Zusammenhang mit medizinischen Produkten oder Produkten für die *in vitro* Diagnostik wird unter Stabilität üblicherweise verstanden, dass ein Produkt über die Dauer seiner Lagerung oder seines Gebrauchs seine erforderlichen Spezifikationen einhält, beziehungsweise im günstigsten Fall die Eigenschaften und Charakteristika beibehält wie zum Zeitpunkt seiner Produktion.

Zur Vorhersage der Stabilität einer Proteinzubereitung werden häufig beschleunigte Stabilitätssstudien (accelerated stability studies) durchgeführt. Diese Studien sind so ausgelegt, dass der chemische oder physikalische Abbau eines Proteinprodukts unter erhöhten Stressbedingungen (z. B. erhöhte Temperatur, hohe Luftfeuchte, Licht, Erschütterungen) beschleunigt wird. Aus den Stabilitätsuntersuchungen unter erhöhten Stressbedingungen können Rückschlüsse auf die tatsächliche Langzeitstabilität (long term stability) eines Proteins gezogen werden. Dies kann auf der Grundlage von Erfahrungen mit ähnlichen Proteinprodukten und/oder unter Verwendung der Arrhenius-Gleichung oder anderer festgelegter mathematischer Modelle erfolgen.

Gewöhnlich ist die Geschwindigkeit der Abbauvorgänge in Proteinlösungen unter den echten Lagerungsbedingungen (z. B. +2 bis +8 ˚C) langsam. Bei erhöhten Temperaturen nehmen die Bewegungen von Molekülen und ihre Schwingungsamplituden zu. Dadurch kommt es zu häufigeren Molekülkollisionen und infolgedessen auch zu stärkerer Moleküldegradation. Die Beziehung zwischen Reaktionsgeschwindigkeit und Temperatur wurde von van't Hoff in der Reaktionsgeschwindigkeit-Temperatur-Regel (RGT-Regel) zusammengefasst. Diese Regel besagt, dass eine Temperaturerhöhung um 10 Kelvin eine Steigerung der Reaktionsgeschwindigkeit um das zwei- bis vierfache zur Folge hat. Mathematisch und physikalisch wird dieses Phänomen über die Arrhenius-Gleichung beschrieben, nach der die Temperaturabhängigkeit der Reaktionsgeschwindigkeit eine Exponentialfunktion ist.

Die Zugabe stabilisierender Agenzien zu einer Proteinformulierung ist häufig das Mittel der Wahl, um die Stabilität eines Proteins zu verbessern. Allerdings ist es bislang nicht gelungen eine universelle Stabilisierungsstrategie zu entwickeln, die für alle Proteine angewendet werden kann. Es wird spekuliert, dass der protektive Effekt einer stabilisierenden Substanz abhängig ist von den spezifischen strukturellen Eigenschaften des zu stabilisierenden Proteins. Aufgrund dieses Phänomens werden stabilisierende Substanzen für bestimmte Proteine vornehmlich anhand empirischer Studien ausgewählt. Als mögliche Stabilisatoren werden unter anderem Antioxidanzien oder reduzierende Agenzien zur Verhinderung von oxidativem Abbau, Proteinase-Inhibitoren zur Verhinderung von proteolytischen Prozessen, chelatisierende Agenzien zum Ausschluss von Schwermetallionen oder Bakteriostatika und Fungizide zur Vermeidung von mikrobiellem Wachstum verwendet. Aktivitätsverluste durch physikalische Effekte wie Adsorption, Denaturierung durch Oberflächen, Hitzedenaturierung, Trocknung, wiederholtes Einfrieren und Auftauen können vielfach durch Zugabe von Glycerin, Kohlenhydraten, Aminosäuren, hydrophilen Polymeren oder inerten Proteinen deutlich reduziert werden. Humanes Serumalbumin (HSA), bovines Serumalbumin (BSA) oder Ovalbumin werden oft als stabilisierende Agenzien für gefriergetrocknete Proteine verwendet. Ein Nachteil bei der Verwendung derartiger Proteinzusätze ist die mögliche Kontamination mit biologisch aktiven Materialien, wie z. B. Proteasen oder Proteaseinhibitoren, die die biologische Aktivität des Proteins, das eigentlich stabilisiert werden soll, negativ beeinflusst. Darüber hinaus schließt die Zugabe großer Mengen von Albuminen in den meisten Fällen die Möglichkeit einer anschließenden physikochemischen Analyse des gewünschten Proteins aus.

US-B2-6514940 offenbart ein flussiges Antithrombin III-Präparat, das von Stabilisatoren (z.B. Saccharose, Aminosäuren, und zusäztlich Ammoniumsulfat) geschützt ist.

Der vorliegenden Erfindung lag die Aufgabe zugrunde, ein Verfahren zur Stabilisierung von Serin-Endopeptidasen bereitzustellen.

Bei einer Serin-Endopeptidase handelt es sich im Sinne der vorliegenden Erfindung um ein Enzym, das die folgenden strukturellen bzw. funktionellen Merkmale aufweist:

I. Bei einer Serin-Endopeptidase handelt es sich immer um eine Hydrolase, die Peptidbindungen spaltet;
II. diese Aktivität hängt ab von einer Gruppe von Aminosäureresten, die bezogen auf die Primärstruktur des Enzyms weit entfernt voneinander liegen können, durch eine höherrangige Struktur jedoch im aktiven, katalytischen Zentrum angenähert sind ("katalytische Triade"), wobei einer dieser Aminosäurereste immer ein Serin-Rest ist;
III. im Gegensatz zu den so genannten Exopeptidasen, die ein Polypeptid vom C- oder N-Terminus her spalten und je nach Spezifität Tripeptide, Dipeptide oder auch einzelne Aminosäuren freisetzen, spaltet eine Serin-Endopeptidase Peptidbindungen, die sich innerhalb eines Proteins oder Polypeptids befinden;
IV. eine Serin-Endopeptidase wird durch Phenylmethylsulfonylfluorid (PMSF) irreversibel inhibiert, da PMSF den Serin-Rest im aktiven Zentrum sulfonyliert;
V. eine Serin-Endopeptidase wird durch Düsopropylfluorophosphat (DFP) irreversibel inhibiert, da DFP den Serin-Rest im aktiven Zentrum phosphoryliert.

Gemäß der internationalen Enzymklassifikation (Enzyme Classification = E.C.), die von der International Union of Pure and Applied Chemistry (IUPAC) und der International Union of Biochemistry geschaffen wurde, wird jedem Enzym eine aus vier Zahlen bestehende E.C.-Nummer zugewiesen (siehe auch www.chem.qmul.ac.uk/iubmb/enzyme). Die Serin-Endopeptidasen, die die obengenannten Merkmale aufweisen, sind in der Enzymklasse E.C.3.4.21 zusammengefasst. Die einzelnen Mitglieder dieser Klasse erhalten jeweils eine zusätzliche Nummer. In Tabelle 1 sind die gegenwärtig bekannten Mitglieder der Enzymklasse der Serin-Endopeptidasen E.C. 3.4.21 aufgelistet.

**Tabelle 1**

| | |
|---|---|
| E.C.3.4.21.1 | Chymotrypsin |
| E.C.3.4.21.2 | Chymotrypsin C |
| E.C.3.4.21.3 | Metridin |
| E.C.3.4.21.4 | Trypsin |
| E.C.3.4.21.5 | Thrombin |
| E.C.3.4.21.6 | Blutgerinnungsfaktor Xa |
| E.C.3.4.21.7 | Plasmin |
| E.C.3.4.21.9 | Enteropeptidase |
| E.C.3.4.21.10 | Acrosin |
| E.C.3.4.21.12 | Alpha-lytische Endopeptidase |
| E.C.3.4.21.19 | Glutamyl Endopeptidase |
| E.C.3.4.21.20 | Cathepsin G |
| E.C.3.4.21.21 | Blutgerinnungsfaktor VIIa |
| E.C.3.4.21.22 | Blutgerinnungsfaktor IXa |
| E.C.3.4.21.25 | Cucumisin |
| E.C.3.4.21.26 | Prolyl-Oligopeptidase |
| E.C.3.4.21.27 | Blutgerinnungsfaktor XIa |
| E.C.3.4.21.32 | Brachyurin |
| E.C.3.4.21.34 | Plasma-Kallikrein |
| E.C.3.4.21.35 | Gewebs-Kallikrein |
| E.C.3.4.21.36 | Pankreas-Elastase |
| E.C.3.4.21.37 | Leukozyten-Elastase |
| E.C.3.4.21.38 | Blutgerinnungsfaktor XIIa |
| E.C.3.4.21.39 | Chymase |

(fortgesetzt)

| | |
|---|---|
| E.C.3.4.21.41 | Komplementfaktor C1 r |
| E.C.3.4.21.42 | Komplementfaktor C1s |
| E.C.3.4.21.43 | C3/C5 Konvertase (klassisch) |
| E.C.3.4.21.45 | Komplementfaktor I |
| E.C.3.4.21.46 | Komplementfaktor D |
| E.C.3.4.21.47 | C3/C5 Konvertase (alternativ) |
| E.C.3.4.21.48 | Cerevisin |
| E.C.3.4.21.49 | Hypodermin C |
| E.C.3.4.21.50 | Lysosymale Endopeptidase |
| E.C.3.4.21.53 | Endopeptidase La |
| E.C.3.4.21.54 | Gamma-Renin |
| E.C.3.4.21.55 | Venombin Ab |
| E.C.3.4.21.57 | Leucyl-Endopeptidase |
| E.C.3.4.21.59 | Tryptase |
| E.C.3.4.21.60 | Scutelarin |
| E.C.3.4.21.61 | Kexin |
| E.C.3.4.21.62 | Subtilisin |
| E.C.3.4.21.63 | Oryzin |
| E.C.3.4.21.64 | Proteinase K |
| E.C.3.4.21.65 | Thermomycolin |
| E.C.3.4.21.66 | Thermitase |
| E.C.3.4.21.67 | Endopeptidase So |
| E.C.3.4.21.68 | Gewebs-Plasminogen-Aktivator |
| E.C.3.4.21.69 | Protein C (aktiviert) |
| E.C.3.4.21.70 | Pankreatische Endopeptidase E |
| E.C.3.4.21.71 | Pankreatische Elastase II |
| E.C.3.4.21.72 | IgA-spezifische Serin-Endopeptidase |
| E.C.3.4.21.73 | Urokinase-Plasminogen-Aktivator |
| E.C.3.4.21.74 | Venombin A |
| E.C.3.4.21.75 | Furin |
| E.C.3.4.21.76 | Myeloblastin |
| E.C.3.4.21.77 | Semenogelase |
| E.C.3.4.21.78 | Granzym A |
| E.C.3.4.21.79 | Granzym B |
| E.C.3.4.21.80 | Streptogrisin A |
| E.C.3.4.21.81 | Streptogrisin B |
| E.C.3.4.21.82 | Glutamyl-Endopeptidase II |
| E.C.3.4.21.83 | Oligopeptidase B |
| E.C.3.4.21.84 | Limulus Gerinnungsfaktor C |
| E.C.3.4.21.85 | Limulus Gerinnungsfaktor B |
| E.C.3.4.21.86 | Limulus Gerinnungsenzym |
| E.C.3.4.21.87 | Omptin |
| E.C.3.4.21.88 | Repressor LexA |
| E.C.3.4.21.89 | Signalpeptidase I |
| E.C.3.4.21.90 | Togavirin |
| E.C.3.4.21.91 | Flavirin |
| E.C.3.4.21.92 | Endopeptidase Clp |
| E.C.3.4.21.93 | Proprotein-Konvertase 1 |
| E.C.3.4.21.94 | Proprotein-Konvertase 2 |
| E.C.3.4.21.95 | Schlangengift Faktor V Aktivator |
| E.C.3.4.21.96 | Lactocepin |

(fortgesetzt)

| | |
|---|---|
| E.C.3.4.21.97 | Assemblin |
| E.C.3.4.21.98 | Hepacivirin |
| E.C.3.4.21.99 | Spermosin |
| E.C.3.4.21.100 | Pseudomonapepsin |
| E.C.3.4.21.101 | Xanthomonapepsin |
| E.C.3.4.21.102 | C-terminal prozessierende Peptidase |
| E.C.3.4.21.103 | Physarolisin |

Die Mehrzahl der Serin-Endopeptidasen ist tierischen bzw. humanen Ursprungs, wird sezerniert und verfügt über ein N-terminales Signalpeptid. Die Serin-Endopeptidasen, die über ein solches N-terminales Signalpeptid verfügen, werden zunächst als Vorläufer mit N-terminalem Propeptid synthetisiert. Im Zuge der Aktivierung einer solchen Serin-Endopeptidase wird das N-terminale Propetid abgespalten, wobei nicht immer eine vollständige Abspaltung des Propetids zur Aktivierung notwendig ist, sondern in manchen Fällen das Propeptid nach seiner Spaltung über DisulfidBrücken an die schwere Kette der Protease gebunden bleibt. Nichtsdestotrotz führt die Spaltung des Vorläufers zu einer strukturellen Änderung innerhalb des Proteinmoleküls, wodurch das katalytische Zentrum der Protease in den aktiven Zustand überführt wird.

Zu den Serin-Endopeptidasen gehören unter anderem auch die Blutgerinnungsfaktoren Faktor II (F II), Faktor VII (F VII), Faktor IX (F IX), Faktor X (F X), Faktor XI (F XI) und Faktor XII (F XII). In der aktivierten Form werden die Faktoren durch den Anhang "a" kenntlich gemacht: Faktor IIa (F IIa, Thrombin), Faktor VIIa (F VIIa), Faktor IXa (F IXa), Faktor Xa (F Xa), Faktor XIa (F XIa) und Faktor XIIa (F XIIa). Am Beispiel der Blutgerinnungsfaktoren soll im Folgenden die vorliegende Erfindung veranschaulicht werden, ohne dass jedoch der Umfang der Erfindung auf diese Gruppe beschränkt sein soll.

Zubereitungen isolierter oder angereicherter Blutgerinnungsfaktoren werden sowohl für therapeutische als auch für diagnostische Zwecke benötigt. Bei der Behandlung von Erkrankungen, die auf einen angeborenen oder erworbenen Mangel an einem oder mehreren Blutgerinnungsfaktoren zurückgehen, werden dem Patienten häufig Substitutionspräparate injiziert, die den oder die fehlenden Blutgerinnungsfaktoren in konzentrierter Form enthalten. In diesem Fall sind die Blutgerinnungsfaktoren Bestandteil einer pharmazeutisch verträglichen Zubereitung. Im diagnostischen Bereich werden Blutgerinnungsfaktoren, bevorzugt aktivierte Blutgerinnungsfaktoren als Bestandteil von Reagenzien verwendet, welche in Testverfahren zur quantitativen oder qualitativen Bestimmung einer biologischen Aktivität oder eines Analyten in einer Patientenprobe benötigt werden. Als Beispiele sind Reagenzien zu nennen, die F Xa oder Thrombin enthalten, welche in chromogenen Testverfahren zur Bestimmung von Antithrombin oder Heparin zum Einsatz kommen.

In Bezug auf Testkits, Reagenzien oder therapeutisch einsetzbare Produkte ist es besonders erstrebenswert, die einzelnen Komponenten eines Testkits, die Reagenzien oder die Produkte, die für die therapeutische Anwendung vorgesehen sind, als gebrauchsfertige Flüssigzubereitungen bereitzustellen, da dadurch zusätzliche Arbeitsschritte, wie z. B. die Rekonstitution lyophilisierter Produkte, vermieden werden und außerdem Fehlerquellen verringert werden, wie z. B. die unzureichende Lösung einer Substanz, die Verwendung eines falschen Lösungsmittels oder -volumens, sowie Kontaminationen bei der Herstellung von Lösungen oder Suspensionen aus gefriergetrocknetem Pulver, die sich negativ auf die Qualität und die Sicherheit des gesamten Testverfahrens auswirken können. Nicht zu unterschätzen ist auch das gesundheitliche Risiko für Patienten, das durch die Verabreichung eines falsch rekonstitutierten Therapeutikums entstehen kann. Ein besonders wichtiges Kriterium bei der Entwicklung von flüssigen Testreagenzien oder flüssiger pharmazeutischer Produkte ist eine möglichst lange Haltbarkeit (mindestens mehrere Monate) im flüssigen Zustand unter Lagerungsbedingungen wie z. B. bei Raumtemperatur zwischen +15 und +25 ˚C oder bei Kühlschranktemperaturen zwischen +2 und +8 ˚C.

Besonders problematisch ist die Bereitstellung von gebrauchsfertigen, langzeitstabilen Flüssigzubereitungen, die ein aktives Enzym, wie z. B. einen aktiven Blutgerinnungsfaktor enthalten, da sich viele aktive Enzyme durch eine immanente Instabilität auszeichnen. So ist z. B. der aktivierte F Xa im Vergleich zum inaktiven F X ein labiles Enzym, was dazu führt, dass die katalytische Aktivität von gereinigtem F Xa mit der Lagerungsdauer abnimmt. Aus diesem Grund werden Reagenzien, die einen aktivierten Blutgerinnungsfaktor, wie z. B. Thrombin oder F Xa enthalten, bisher vornehmlich in lyophilisierter Form bereitgestellt und erst kurz vor Gebrauch durch Lösen in einem passenden Lösungsmittel, wie Wasser oder Puffer, rekonstituiert, oder sie werden im tiefgefrorenen Zustand aufbewahrt und erst kurz vor Gebrauch aufgetaut.

Aus der Literatur sind Blutgerinnungsfaktor-Zubereitungen bekannt, die durch den Zusatz verschiedener Additive stabilisiert sind. So wird beispielsweise für gereinigten bovinen F Xa berichtet, dass für in Wasser gelösten F Xa durch den Zusatz von 50 % (v/v) Glycerin und Lagerung bei -20 ˚C eine Stabilisierung für mindestens fünf Monate erzielt wird, während eine bei +4 °C gelagerte F Xa-Zubereitung in Imidazol-Puffer bereits nach einer Woche nur noch 90 % der ursprünglichen Aktivität aufweist [siehe rechte Spalte auf Seite 7736 in Bajaj, S. P. & Mann, K. G. (1973) Simultaneous purification of bovine prothrombin and Factor X. J. Biol. Chem. 248, 7729-7741]. In DE 43 25 872 C1 wird eine virusinaktivierte F Xa-Präparation beschrieben, die wahlweise mit Saccharose oder mit Humanalbumin versetzt wird, zur Langzeitstabilisierung aber lyophilisiert wird. Über einen Lagerungszeitraum von sechs Wochen bei +37 °C konnte keine Veränderung der Aktivität von F Xa beobachtet werden. In EP 680 764 A2 wird ein Verfahren zur Herstellung virusinaktivierter Proteinpräparationen beschrieben, bei dem die zu stabilisierenden Proteine, wie z. B. F Xa mit Lipidvesikeln assoziiert werden, während auf die Verwendung von stabilisierenden Additiven verzichtet wird. Das Patentdokument EP 1 153 608 A1 beschreibt eine Proteinlösung, die ein oder mehrere Blutgerinnungsfaktoren enthält und die durch den Zusatz von Stabilisatoren gegen einen Wirkungsverlust bei der Pasteurisierung geschützt ist. Als stabilisierend wird der Zusatz von Sacchariden und/oder von Aminosäuren aus der Gruppe Arginin, Lysin, Histidin, Phenylalanin, Tryptophan, Tyrosin, Asparaginsäure und ihren Salzen und Glutaminsäure und ihren Salzen beschrieben.

Der vorliegenden Erfindung lag die Aufgabe zugrunde eine im flüssigen Zustand langzeitstabile Zubereitung bereitzustellen, die eine Serin-Endopeptidase enthält. Die Lösung der Aufgabe besteht in der Bereitstellung der in den Ansprüchen beschriebenen erfindungsgemäßen Verfahren und Gegenstände.

Gegenstand der vorliegenden Erfindung ist eine Zubereitung, die mindestens eine Serin-Endopeptidase enthält, wobei die Serin-Endopeptidase in der Zubereitung in gewünschter Reinheit und Konzentration enthalten ist. Bevorzugterweise handelt es sich bei der in der Zubereitung enthaltenen Serin-Endopeptidase um eine gereinigte Serin-Endopeptidase. Die Gewinnung einer gereinigten Serin-Endopeptidase kann durch ein beliebiges, dem Fachmann als geeignet erscheinendes Verfahren erfolgt sein, das die Reinigung der Serin-Endopeptidase aus dem organischen Rohmaterial ermöglicht, in welchem die Serin-Endopeptidase natürlicherweise vorkommt oder gentechnisch erzeugt wurde. Je nach gewünschter Reinheit der Serin-Endopeptidase können Reinigungsverfahren angewandt werden, die die Abtrennung von Begleitstoffen wie Kohlenhydraten, Lipiden, Nukleinsäuren, Proteinen und/oder anderen Biomolekülen ermöglichen. Rohmaterialien für die Gewinnung einer Serin-Endopeptidase können z. B. tierische oder humane Gewebe oder Körperflüssigkeiten (z. B. Blut, Plasma, Serum, Lymphflüssigkeit) sein, Überstände oder Lysate tierischer oder humaner Zellkulturen, oder Kulturen eukaryontischer Zellen oder von Mikroorganismen, wie Bakterien oder Pilzen, die eine rekombinante Serin-Endopeptidase exprimieren. Beispiele für Verfahren, die bekanntermaßen zur Reinigung von Proteinen verwendet werden, sind chromatographische Trennverfahren, wie die Ionenaustausch-, die Gelfiltrations-, die hydrophobe Interaktions- oder die Affinitätschromatographie. Daneben können auch die präparative Gelelektrophorese, die präparative isoelektrische Fokussierung, die Chromatofokussierung, die Fällung und die Ultrazentrifugation für die Reinigung von Proteinen aus einem Proteinextrakt verwendet werden.

Die vorliegende Erfindung betrifft eine Zubereitung, die mindestens eine Serin-Endopeptidase und zusätzlich entweder a) Ammoniumacetat ($CH_3COONH_4$) oder b) mindestens eine Polyaminosäure oder c) Glycerin zusammen mit mindestens einer Aminosäure aus der Gruppe Asparaginsäure und deren Salze, Glutaminsäure und deren Salze, Histidin und Glycin, oder d) eine beliebige Kombination der Zusätze a), b) und c) enthält.

Es wurde gefunden, dass der alleinige Zusatz von a) Ammoniumacetat, der alleinige Zusatz von b) mindestens einer Polyaminosäure, der alleinige Zusatz von c) Glycerin zusammen mit mindestens einer Aminosäure aus der Gruppe Asparaginsäure und deren Salze, Glutaminsäure und deren Salze, Histidin und Glycin sowie der Zusatz einer Kombination der Additive a) und b), oder a) und c), oder b) und c), oder a) und b) und c) eine Stabilisierung der Serin-Endopeptidase bewirkt. Überraschenderweise verfügt eine erfindungsgemäße Zubereitung im flüssigen Zustand über eine höhere Stabilität und somit längere Lagerungsfähigkeit als eine entsprechende Zubereitung in Abwesenheit der genannten Additive.

Zur Bestimmung der Stabilität einer erfindungsgemäßen Zubereitung wurden Stabilitätsstudien unter beschleunigten Stressbedingungen durchgeführt (siehe z. B. Beispiel 1). Untersucht wurde die Stabilität einer Zubereitung im flüssigen Zustand bei einer Temperatur von +52 °C. Als Stabilität wird hier die Erhaltung der biologischen Aktivität einer Zubereitung bezeichnet. Die Stabilität einer Zubereitung ist umso höher je geringer der Verlust der biologischen Aktivität der enthaltenen Serin-Endopeptidase oder je geringer die Anzahl an Degradationsprodukten der enthaltenen Serin-Endopeptidase ist.

Es konnte beobachtet werden, dass z. B. die Serin-Endopeptidase F Xa in einer erfindungsgemäßen Zubereitung im flüssigen Zustand über einen Zeitraum von 48 Stunden bei einer Lagerungstemperatur von +52 °C einen Aktivitätsverlust von weniger als 50 % aufweist. Mit Hilfe der Arrhenius-Gleichung kann anhand der Stabilität der Zubereitung bei +52 °C die zu erwartende Stabilität der Zubereitung bei anderen Temperaturen grob abgeschätzt werden. Nach der Arrhenius-Gleichung ist die Stabilität einer Probe bei +2 °C gegenüber der Stabilität bei +52 °C grob geschätzt etwa 32-fach gesteigert, d. h. dass z. B. Aktivitäten, die nach zweitägiger Inkubation bei +52 °C gemessen werden, bei einer Lagerungstemperatur von nur +2°C noch nach etwa 64 Tagen erwartet werden können. Zur Bestimmung des Aktivitätsverlustes wird vorzugsweise die Aktivität der in der Zubereitung enthaltenen Serin-Endopeptidase bestimmt und zwar einmal zum Zeitpunkt $t_0$, d. h. unmittelbar nach Herstellung der Flüssigzubereitung und mindestens ein weiteres Mal nach Lagerung der Flüssigzubereitung bei +52 °C über einen definierten Zeitraum, vorzugsweise über einen Zeitraum von 48 Stunden. Der Aktivitätsverlust ergibt sich durch den Vergleich der gemessenen Aktivität zum Zeitpunkt $t_0$ (entspricht 100 %) mit der Aktivität, die nach Lagerung der Flüssigzubereitung bei +52 °C noch gemessen wird. Zur Bestimmung der Stabilität einer Zubereitung, die beispielsweise F Xa in angereicherter Form enthält, eignet sich z. B. ein Verfahren, bei dem die proteolytische Aktivität von F Xa anhand der Spaltung eines chromogenen Peptidsubstrats gemessen wird (siehe Beispiel 1).

Eine bevorzugte Ausführungsform der Erfindung betrifft eine Zubereitung, die mindestens eine Serin-Endopeptidase und zusätzlich Ammoniumacetat zur Stabilisierung enthält. Eine solche Zubereitung kann zusätzlich Glycerin oder zusätzlich mindestens eine Aminosäure aus der Gruppe Asparaginsäure und deren Salze, Glutaminsäure und deren Salze, Histidin und Glycin enthalten.

Eine andere Ausführungsform der Erfindung betrifft eine Zubereitung in der mindestens eine Serin-Endopeptidase und zusätzlich mindestens eine Polyaminosäure zur Stabilisierung enthalten ist. Eine solche Zubereitung kann zusätzlich Glycerin oder zusätzlich mindestens eine Aminosäure aus der Gruppe Asparaginsäure und deren Salze, Glutaminsäure und deren Salze, Histidin und Glycin enthalten.

Sofern einer erfindungsgemäßen Zubereitung Ammoniumacetat zugesetzt ist, ist das Ammoniumacetat vorzugsweise in einer Endkonzentration von 25 bis 1000 mM, bevorzugt 400 bis 1000 mM, besonders bevorzugt von 700 bis 1000 mM enthalten.

Sofern einer erfindungsgemäßen Zubereitung eine Polyaminosäure zugesetzt ist, kann es sich vorzugsweise um eine Polyaminosäure aus der Gruppe Poly-L-Glutamat und Poly-L-Aspartat handeln. Eine Polyaminosäure ist vorzugsweise in einer Endkonzentration von 1 bis 10 mM, bevorzugt von 2 bis 10 mM, besonders bevorzugt von 2 bis 5 mM enthalten.

Verschiedene Ausführungsformen der erfindungsgemäßen Zubereitung können Glycerin enthalten. Sofern einer erfindungsgemäßen Zubereitung Glycerin zugesetzt ist, ist das Glycerin vorzugsweise in einer Endkonzentration von 0,5 bis 50 Volumenprozent, bevorzugt von 10 bis 50 Volumenprozent, besonders bevorzugt von 30 bis 50 Volumenprozent enthalten.

Weitere Ausführungsformen der erfindungsgemäßen Zubereitung können eine oder mehrere Aminosäuren aus der Gruppe Asparaginsäure und deren Salze, Glutaminsäure und deren Salze, Histidin und Glycin enthalten. Sofern einer erfindungsgemäßen Zubereitung eine Aminosäure aus der Gruppe Histidin und Glycin zugesetzt ist, ist Histidin oder Glycin vorzugsweise in einer Endkonzentration von 10 bis 250 mM, bevorzugt von 25 bis 200 mM, besonders bevorzugt von 100 bis 150 mM enthalten. Sofern einer erfindungsgemäßen Zubereitung eine Aminosäure aus der Gruppe Asparaginsäure und deren Salze und Glutaminsäure und deren Salze enthält, ist die Aminosäure oder deren Salz vorzugsweise in einer Endkonzentration von 10 bis 1000 mM, bevorzugt von 400 bis 1000 mM, besonders bevorzugt von 500 bis 800 mM enthalten.

Überraschenderweise wurde gefunden, dass der Zusatz der Aminosäure Lysin zu einer Zubereitung, die eine Serin-Endopeptidase enthält, im Gegensatz zu den vorgenannten Aminosäuren destabilisierend wirkt und zwar in jeder Kombination mit einem oder mehreren der anderen erfindungsgemäßen Additive. Besonders zu bevorzugenden Ausführungsformen der erfindungsgemäßen Zubereitungen ist daher kein Lysin zugesetzt.

Andere Ausführungsformen der erfindungsgemäßen Zubereitung können zusätzlich einen oder mehrere nicht-reduzierende Zucker enthalten, bevorzugterweise aus der Gruppe Saccharose und Trehalose. Sofern einer erfindungsgemäßen Zubereitung ein nicht-reduzierender Zucker zugesetzt ist, ist der Zucker vorzugsweise in einer Endkonzentration von 20 bis 500 mM, bevorzugt von 50 bis 400 mM, besonders bevorzugt von 200 bis 300 mM enthalten.

Einer erfindungsgemäßen Zubereitung können überdies noch weitere Additive zugesetzt sein, wie z. B. Polyethylenglykol, Polyethylenimin, ionische oder nichtionische Detergenzien (z. B. Triton® X-100, Tween® 20, Brij® 35), Proteaseinhibitoren, Salze, wie z. B. $Ca^{2+}$-Ionen, Heparine, Albumine, Konservierungsmittel mit bakterizider, fungizider oder algizider Wirkung (z. B. Natriumazid, Kathon®, Mergal® etc.) und andere, sofern die Anwesenheit eines solchen weiteren Bestandteils die Stabilität der erfindungsgemäßen Zubereitung nicht vermindert oder die Verwendung der Zubereitung zu einem spezifischen Zweck beeinträchtigt. So ist es insbesondere bei einer erfindungsgemäßen Zubereitung, die für eine therapeutische Verwendung vorgesehen ist, notwendig auf pharmazeutisch unverträgliche Zusätze zu verzichten.

Der pH-Wert einer erfindungsgemäßen Zubereitung kann zwischen 6,5 und 9,5 liegen, bevorzugt zwischen 7,4 und 8,5 und beträgt besonders bevorzugterweise 8,0.

Bevorzugte Ausführungsformen der erfindungsgemäßen Zubereitung enthalten eine Serin-Endopeptidase aus der Gruppe der tierischen oder humanen Blutgerinnungsfaktoren, insbesondere einen Blutgerinnungsfaktor aus der Gruppe F II, F VII, F IX, FX, F XI und F XII bzw. aus der Gruppe der aktivierten Blutgerinnungsfaktoren F IIa, F VIIa, F IXa, FXa, F XIa und F XIIa. Weiterhin bevorzugt sind Blutgerinnungsfaktoren bovinen Ursprungs.

Andere Ausführungsformen der erfindungsgemäßen Zubereitung enthalten eine Serin-Endopeptidase aus der Gruppe der tierischen oder humanen Komplementfaktoren umfassend Komplementfaktor C1r, C1s, Komplementfaktor D und Komplementfaktor I.

Andere Ausführungsformen der erfindungsgemäßen Zubereitung enthalten eine oder mehrere der Serin-Endopeptidasen, die in Tabelle 1 aufgelistet sind. Besonders bevorzugte Ausführungsformen enthalten eine Serin-Endopeptidase aus der Gruppe Chymotrypsin, Trypsin, Plasmin, Acrosin, Cathepsin G, Plasma-Kallikrein, Gewebs-Kallikrein, Pankreas-Elastase, Leukozyten-Elastase, C3/C5 Konvertase (klassisch), C3/C5 Konvertase (alternativ), Subtilisin, Proteinase K, aktiviertes Protein C, Gewebs-Plasminogen-Aktivator, Urokinase-Plasminogen-Aktivator, Furin, Limulus Gerinnungsfaktor C, Limulus Gerinnungsfaktor B, Limulus Gerinnungsenzym und Schlangengift Faktor V Aktivator.

Aufgrund der stabilisierenden Wirkung der erfindungsgemäßen Additive wird eine erfindungsgemäße Zubereitung vorzugsweise in flüssiger Form bereitgestellt. Nichtsdestotrotz ist es möglich, eine erfindungsgemäße Zubereitung zu lyophilisieren. Zu diesem Zweck können gegebenenfalls weitere Stabilisatoren mit kryoprotektiver Wirkung der erfindungsgemäßen Zubereitung zugesetzt werden, wie z. B. Polysaccharide wie Mannitol, oder Proteine wie Serumalbumine, oder Polygelin, ein Gelatinederivat, oder Polyole.

Ein weiterer Gegenstand der vorliegenden Erfindung betrifft Verfahren zur Herstellung einer erfindungsgemäßen stabilisierten Zubereitung, die eine Serin-Endopeptidase in angereicherter Form enthält bzw. Verfahren zur Stabilisierung einer eine Serin-Endopeptidase enthaltenden Zubereitung. Darunter ist jedes Verfahren zu verstehen, das sicherstellt, dass einer Zubereitung, die eine Serin-Endopeptidase enthält, zusätzlich entweder a) Ammoniumacetat ($CH_3COONH_4$) oder b) mindestens eine Polyaminosäure oder c) Glycerin zusammen mit mindestens einer Aminosäure aus der Gruppe Asparaginsäure und deren Salze, Glutaminsäure und deren Salze, Histidin und Glycin oder d) eine beliebige Kombination der Zusätze a), b) und c) zugesetzt wird. In einer bevorzugten Ausführungsform wird dazu eine wässrige Lösung, die eine Serin-Endopeptidase enthält, mit einer oder mehreren Lösungen vermischt, die jeweils eines oder mehrere der erfindungsgemäßen Additive enthalten. In einer anderen bevorzugten Ausführungsform wird dazu eine wässrige Lösung, die eine Serin-Endopeptidase enthält, mit einem oder mehreren löslichen Feststoffen vermischt, die eines oder mehrere der erfindungsgemäßen Additive enthalten. In einer anderen Ausführungsform kann ein Lyophilisat, das die Serin-Endopeptidase enthält, in einem Rekonstitutionsmedium gelöst werden, das bereits eines oder mehrere der erfindungsgemäßen Additive enthält.

Die vorliegende Erfindung betrifft weiterhin die Verwendung von a) Ammoniumacetat und/oder von b) Polyaminosäuren und/oder von c) Glycerin in Kombination mit mindestens einer Aminosäure aus der Gruppe Asparaginsäure und deren Salze, Glutaminsäure und deren Salze, Histidin und Glycin zur Stabilisierung einer Zubereitung, die eine Serin-Endopeptidase enthält.

Ein weiterer Gegenstand der vorliegenden Erfindung betrifft die Verwendung einer erfindungsgemäßen stabilisierten Zubereitung, die eine Serin-Endopeptidase enthält, in einem analytischen oder diagnostischen Verfahren oder in einem biokatalytischen Herstellungsverfahren oder zu therapeutischen Zwecken. Eine bevorzugte Verwendung betrifft die Verwendung einer erfindungsgemäßen stabilisierten Zubereitung als Testreagenz in einem Testverfahren zur Bestimmung eines Analyten oder zur Bestimmung einer biologischen Aktivität, wie z. B. des Gerinnungspotenzials einer Blut- oder Plasmaprobe anhand einer Gerinnungszeit.

Eine Ausführungsform der vorliegenden Erfindung betrifft die Verwendung einer stabilisierten Zubereitung, die Subtilisin enthält, in einem Verfahren zur Herstellung von Waschmitteln, zur Herstellung von Tiernahrung, in einem Verfahren zur Lederherstellung oder zur Racematspaltung in einem organischen Syntheseverfahren.

Eine weitere Ausführungsform der vorliegenden Erfindung betrifft die Verwendung einer stabilisierten Zubereitung, die Proteinase K enthält, in einem Verfahren zum Abbau von Proteinen in Zelllysaten und/oder zur Freisetzung von Nukleinsäuren aus Zellen oder Gewebe oder in einem Verfahren zur Identifizierung von Prionenprotein (Scrapie etc.).

Eine weitere Ausführungsform der vorliegenden Erfindung betrifft die Verwendung einer stabilisierten Zubereitung, die Furin enthält, in einem Verfahren zur Aktivierung von enzymatisch inaktiven Vorstufen proteolytischer Enzyme (Zymogene).

Eine bevorzugte Verwendung einer erfindungsgemäßen stabilisierten Zubereitung, die mindestens einen tierischen oder humanen Blutgerinnungsfaktor, bevorzugt aus der Gruppe F II, F VII, F IX, F X, F XI und F XII und/oder aus der Gruppe F IIa, F VIIa, F IXa, F Xa, F XIa und F XIIa enthält, ist die Verwendung als Testreagenz in einem Testverfahren zur Bestimmung von Gerinnungs- und/oder Fibrinolyseparametern in Blut- oder Plasmaproben. Ein Beispiel für eine derartige Verwendung einer Zubereitung als Testreagenz ist die Verwendung einer erfindungsgemäßen Zubereitung, die F Xa oder F IIa (Thrombin) enthält. F Xa-enthaltende Reagenzien werden in verschiedenen Testverfahren der Gerinnungsdiagnostik verwendet, wie z. B. in Testverfahren zur Bestimmung von Antithrombin oder von Heparin in Patientenproben. Ausführungsformen F Xa-basierter Testverfahren sind z. B. in den Patentdokumenten EP 216 179 B1 (Beispiel 2) und US 5,308,755 (Example 2) beschrieben. Diesen Verfahren liegt das allgemeine Testprinzip zugrunde, dass eine zu untersuchende Patientenprobe u. a. mit einem Überschuss F Xa versetzt wird und die F Xa-hemmende Wirkung von dem in der Probe enthaltenen Antithrombin oder Heparin bestimmt wird, indem nach einer Inkubationsphase die Restaktivität des F Xa bestimmt wird, beispielsweise mit einem chromogenen Substrat. Auch F IIa (Thrombin)-enthaltende Reagenzien werden in verschiedenen Testverfahren der Gerinnungsdiagnostik verwendet, wie z. B. in Testverfahren zur Bestimmung der Thrombinzeit, des gerinnbaren Fibrins nach Clauss oder auch in Testverfahren zur Bestimmung von Antithrombin oder Heparinkofaktor 11. Diese Testverfahren beruhen im Wesentlichen darauf, dass einer zu untersuchenden Patientenprobe eine definierte Menge F IIa (Thrombin) zugesetzt wird und entweder die Fibrinbildung in Form einer Gerinnungszeit gemessen wird oder die Spaltung eines Thrombinsubstrats gemessen wird. Eine Ausführungsform eines F IIa (Thrombin)-basierten Testverfahrens zur Bestimmung von Antithrombin ist z. B. in dem Patentdokument EP 216 179 B1 (Beispiel 1) beschrieben. Aufgrund der mangelnden Stabilität herkömmlicher Faktor Xa- oder F IIa (Thrombin)-Zubereitungen werden F Xa- sowie F IIa (Thrombin)-enthaltende Testreagenzien überwiegend als Lyophilisate bereitgestellt. Aufgrund der guten Stabilität einer erfindungsgemäßen F Xa- oder F IIa (Thrombin)-Zubereitung, ist die Verwendung einer erfindungsgemäßen F Xa-Zubereitung oder F IIa (Thrombin)-Zubereitung als Testreagenz, die in flüssigem Zustand über einen längeren Zeitraum gelagert werden kann, vorteilhaft. Ein anderes Beispiel für eine Verwendung einer Zubereitung als Testreagenz ist die Verwendung einer erfindungsgemäßen Zubereitung, die Chymotrypsin enthält, in einem Verfahren zur Bestimmung von alpha$_1$-Antichymotrypsin, einem Inhibitor zellulärer Proteasen, wie es z. B. in dem Patentdokument EP 216 179 B1 (Beispiel 5) beschrieben ist.

Ein weiterer Gegenstand der vorliegenden Erfindung betrifft die Verwendung einer erfindungsgemäßen stabilisierten Zubereitung, die eine Serin-Endopeptidase enthält, als Kontroll- oder Standardlösung in einem Testverfahren, das zur quantitativen oder qualitativen Bestimmung der Serin-Endopeptidase vorgesehen ist. Ein Beispiel dafür ist die Verwendung einer erfindungsgemäßen stabilisierten Zubereitung als Kontrolle oder Standard, die einen tierischen oder humanen Blutgerinnungsfaktor enthält, bevorzugt aus der Gruppe F II, F VII, FIX, F X, F XI und F XII und/oder aus der Gruppe F IIa, F VIIa, FIXa, F Xa, F XIa und F XIIa, und zwar in einem Testverfahren, das der Bestimmung der Konzentration oder der Aktivität eines dieser Faktoren dient. Beispielsweise kann eine erfindungsgemäße Zubereitung, die F X bzw. F Xa enthält, als Standard oder Kontrolle in einem Verfahren verwendet werden, das für die Bestimmung der Konzentration oder der Aktivität von F X bzw. F Xa in Patientenproben eingesetzt wird. Die Aktivität bzw. die Konzentration, die in der Patientenprobe mittels eines entsprechenden Aktivitätsassays oder Immunoassays bestimmt wurde, wird dann mit der in einem parallelen Ansatz bestimmten Aktivität bzw. Konzentration des Standards oder der Kontrolle verglichen. Der Vergleich der Messwerte der Patientenprobe mit den Messwerten des Standards oder der Kontrolle, welche eine definierte Menge oder Aktivität der Serin-Endopeptidase enthalten, ermöglicht eine Beurteilung der Serin-Endopeptidase in der Patientenprobe.

Ein weiterer Gegenstand der vorliegenden Erfindung betrifft ein Testkit zur Verwendung bei der Durchführung eines diagnostischen Testverfahrens. Ein solches erfindungsgemäßesTestkit, das eine oder mehrere Testkomponenten enthalten kann, zeichnet sich dadurch aus, dass es eine erfindungsgemäße Zubereitung enthält, die eine Serin-Endopeptidase enthält, bevorzugterweise in Form einer Flüssigzubereitung. Vorteilhafterweise enthält ein Testkit zusätzlich mindestens eine weitere Komponente, die zur Durchführung des diagnostischen Testverfahrens erforderlich ist. Im Falle eines chromogenen Testverfahrens, das der Bestimmung eines Inhibitors der Serin-Endopeptidase dient (siehe oben beschriebene F Xa-, Thrombin- und alpha$_1$-Antichymotrypsin-Teste) sind vorzugsweise weitere Reagenzien in einem Testkit enthalten, wie z. B. ein Reagenz enthaltend ein chromogenes Substrat, das von der jeweiligen Protease gespalten wird. Ein Testkit zur Verwendung in einem Verfahren zur Bestimmung von Heparin in einer Patientenprobe kann zusätzlich ein Reagenz enthalten, das Antithrombin enthält. Ein anderes Testkit zur Verwendung in einem Verfahren zur Bestimmung von Antithrombin in einer Patientenprobe kann zusätzlich ein Reagenz enthalten, das Heparin enthält. Weiterhin .kann ein erfindungsgemäßes Testkit z. B. ein oder mehrere Pufferlösungen, ein oder mehrere Kalibrationslösungen, ein oder mehrere Kontrolllösungen enthalten. Testkits, die zur Verwendung in einem Verfahren vorgesehen sind, bei welchem die biologische Aktivität eines Parameters, z. B. eines Proteaseinhibitors, in einer Plasmaprobe bestimmt wird, können vorzugsweise ein oder mehrere Kalibrationsplasmen bzw. ein oder mehrere Kontrollplasmen enthalten, wie z. B. Normalplasma und/oder abnormales Plasma.

Ein weiterer Gegenstand der vorliegenden Erfindung betrifft die Verwendung einer erfindungsgemäßen stabilisierten Zubereitung als Therapeutikum oder zur Herstellung eines Therapeutikums. Bevorzugt ist die Verwendung von Zubereitungen, die mindestens einen tierischen oder humanen Blutgerinnungsfaktor enthalten, bevorzugt aus der Gruppe F II, F VII, FIX, F X, F XI und F XII und/oder aus der Gruppe der entsprechenden aktivierten Blutgerinnungsfaktoren zur Behandlung von Gerinnungsstörungen bzw. zur Herstellung eines entsprechenden Therapeutikums. Eine erfindungsgemäße Zubereitung enthaltend F X und/oder F Xa eignet sich z. B. zur Behandlung von Patienten mit Defekten des Gerinnungssystems bzw. zur Herstellung eines entsprechenden Therapeutikums.

Die folgenden Ausführungsbeispiele dienen der Veranschaulichung des erfindungsgemäßen Verfahrens.

## Figurenbeschreibung

### Figur 1

Figur 1 ist eine graphische Darstellung des stabilisierenden Effekts verschiedener Ammoniumacetatkonzentrationen in einer F Xa-Flüssigzubereitung, die für 48 Stunden bei +52 °C inkubiert wurde. Die in den Proben enthaltene F Xa-Aktivität wurde zu den Zeitpunkten t = 0; 1 h; 6 h; 24 h und 48 h über die Spaltung eines chromogenen F Xa-Substrats bestimmt. Die zum Zeitpunkt t = 0 gemessene Aktivität wurde gleich 100 % gesetzt. Die zu späteren Zeitpunkten gemessenen Aktivitäten wurden dazu in Relation gesetzt. Es ist deutlich zu erkennen, dass bei Anwesenheit von Ammoniumacetat in der F Xa-Flüssigzubereitung die thermische Stabilität von F Xa gegenüber der Kontrolle ohne Ammoniumacetat erhöht ist. Die Erhöhung der Stabilität ist konzentrationsabhängig, d. h. je höher die Ammoniumacetatkonzentration umso größer ist der stabilisierende Effekt.

**Figur 2**

Figur 2 ist eine graphische Darstellung des stabilisierenden Effekts verschiedener Konzentrationen der Polyaminosäuren Polyglutamat bzw. Polyaspartat in einer F Xa-Flüssigzubereitung, die für 48 Stunden bei +52 ˚C inkubiert wurde. Die in den Proben enthaltene F Xa-Aktivität wurde zu den Zeitpunkten t = 0; 1 h; 6 h; 24 h und 48 h gemessen. Die zum Zeitpunkt t = 0 gemessene Aktivität wurde gleich 100 % gesetzt. Die zu späteren Zeitpunkten gemessenen Aktivitäten wurden dazu in Relation gesetzt. Es ist deutlich zu erkennen, dass bei Anwesenheit einer Polyaminosäure in der F Xa-Flüssigzubereitung die thermische Stabilität von F Xa gegenüber der Kontrolle ohne Polyaminosäure erhöht ist. Die Erhöhung der Stabilität ist konzentrationsabhängig, d. h. je höher die Polyaminosäure-konzentration umso größer ist der stabilisierende Effekt.

**Figur 3**

Figur 3 dokumentiert ein Vergleichsexperiment und ist eine graphische Darstellung des destabilisierenden Effekts verschiedener Konzentrationen der Aminosäure Lysin in einer F Xa-Flüssigzubereitung, die für 48 Stunden bei +52 ˚C inkubiert wurde. Es ist deutlich zu erkennen, dass bei Anwesenheit von L-Lysin in der F Xa-Flüssigzubereitung die thermische Stabilität von F Xa gegenüber der Kontrolle ohne L-Lysin verringert ist. Die Verringerung der Stabilität ist konzentrationsabhängig, d. h. je höher die L-Lysinkonzentration umso größer ist der destabilisierende Effekt. Im Gegensatz zu den erfindungsgemäßen stabilisierenden Substanzen hat die Zugabe von L-Lysin zu einer flüssigen F Xa-Präparation nicht nur keine stabilisierende Wirkung, sondern bewirkt sogar das Gegenteil, nämlich eine Destabilisierung von F Xa.

**Figur 4**

Figur 4 ist eine graphische Darstellung des stabilisierenden Effekts verschiedener Ammoniumacetatkonzentrationen in einer Thrombin-Flüssigzubereitung, die für 48 Stunden bei +52 ˚C inkubiert wurde. Die in den Proben enthaltene Thrombin-Aktivität wurde zu den Zeitpunkten t = 0; 1 h; 6 h; 24 h und 48 h gemessen. Die zum Zeitpunkt t = 0 gemessene Aktivität wurde gleich 100 % gesetzt. Die zu späteren Zeitpunkten gemessenen Aktivitäten wurden dazu in Relation gesetzt. Es ist deutlich zu erkennen, dass bei Anwesenheit von Ammoniumacetat in der Thrombin-Flüssigzubereitung die thermische Stabilität von Thrombin gegenüber der Kontrolle ohne Ammoniumacetat erhöht ist. Die Erhöhung der Stabilität ist konzentrationsabhängig, d. h. je höher die Ammoniumacetatkonzentration umso größer ist der stabilisierende Effekt.

**Beispiele**

**Beispiel 1:**

**Herstellung erfindungsgemäßer F Xa-Flüssigzubereitungen durch Zusatz von Ammoniumacetat, Polyaminosäuren oder Glycerin und Aminosäuren und deren Thermostabilität unter Stressbedingungen**

Zur Gewinnung von humanem F X wurde ein Prothrombin-Komplex-Lyophilisat, das aus humanem Plasma hergestellt worden war, in Lösung gebracht, und die Proteine des Prothrombinkomplex-Präparates wurden durch Zugabe von 25 % (w/v) Ammoniumsulfat gefällt. Das präzipitierte Proteingemisch wurde durch Zentrifugation vom Überstand getrennt, resuspendiert und gegen tri-Natriumcitrat-Dihydrat Puffer (4,0 g/L), pH 6,5 dialysiert. Das Dialysat wurde nachfolgend über ein Dextransulfat-Sepharose Material chromatographiert. Der F X wurde mit Hilfe eines Natriumchlorid-Gradienten in tri-Natriumcitrat-Dihydrat Puffer (pH 6,5) von den übrigen Proteinkomponenten des Prothrombinkomplexes getrennt. Die F X enthaltenden Fraktionen wurden vereinigt und gegen das zehnfache Volumen eines 6 g/L Trishydroxymethylaminomethan und 0,6 g/L Calciumchlorid-Dihydrat enthaltenden Puffers (pH 8,0) dialysiert. Die Aktivierung von F X zu F Xa erfolgte durch Zugabe von 5 mg RVV (Russell's Viper Venom) pro Liter Faktor Xa-Pool und anschließender Inkubation bei Raumtemperatur über Nacht. Der so hergestellte F Xa wurde durch Ammoniumsulfatfällung konzentriert und in 30 g/L Trishydroxymethylaminomethan, 60 g/L Natriumchlorid (pH 8,0) aufgenommen. Zur Lagerung wurde die F Xa enthaltende Proteinlösung anschließend lyophilisiert. Das Lyophilisat enthielt 2 U F Xa pro mg, wobei die Einheit 1 U der Enzymmenge entspricht, die in 1 mL eines Normalplasmas enthalten ist.

Jeweils 2 mg des F Xa-Lyophilisats wurden in 5,4 mL Puffer 1 (19 mmol/L Tris/HCl, 67 mmol/L NaCl, 81 mmol/L $CaCl_2$, pH 8,0), bzw. in Puffer 1, der zusätzlich eine oder mehrere erfindungsgemäße stabilisierende Substanzen enthielt, gelöst und anschließend mit 1,35 µL Fragmin®, einem niedermolekularen Heparin (1000 U/mL; Pharmacia, Kalamazoo, USA), und mit 31,7 µL Aprotinin (1,4 mg/ml) versetzt. Die Endkonzentration der Stammlösung von F Xa betrug 0,73 U/mL.

Zur Untersuchung der Stabilität der erfindungsgemäßen Zubereitungen unter beschleunigten Stressbedingungen wurden von jeder der erfindungsgemäßen F Xa-Flüssigzubereitungen, die eine oder mehrere der erfindungsgemäßen stabilisierenden Substanzen in einer bestimmten Konzentration enthielt, jeweils zwei Proben angesetzt. Alle erfindungsgemäßen F Xa-Proben sowie die F Xa-Kontrollproben, die F Xa im gleichen Puffer, aber ohne Zusatz einer erfindungsgemäßen stabilisierenden Substanz enthielten, wurden für mindestens 48 Stunden bei +52 ˚C in einem Thermoblock inkubiert.

Zur Bestimmung der F Xa-Aktivität in einer Probe wurden zum Zeitpunkt t = 0, d. h. unmittelbar nach dem Lösen des F Xa-Lyophilisats in Puffer 1 und vor der Erwärmung auf +52 ˚C und dann nach verschiedenen Inkubationszeiten bei +52 ˚C jeweils 80 µL-Aliquots entnommen, und die F Xa-Aktivität wurde auf dem automatischen Gerinnungsmessgerät Sysmex® CA-7000 (Sysmex Corporation, Kobe, Japan) bestimmt. Dazu wurde das jeweilige Aliquot vom Gerinnungsautomaten zunächst mit 40 µL Puffer 1 verdünnt. Nach einer Inkubation für 12 Sekunden bei +37 ˚C wurden jeder Probe 80 µl Substrat-Reagenz (Z-D-Leu-Gly-Arg-ANBA-methylamid, 1,5 mM) zugegeben und anschließend wurde für weitere 84 Sekunden bei +37 ˚C inkubiert. Die F Xa-abhängige Bildung des Chromophors ANBA (5-Amino-2-Nitro-Benzoesäure) wurde bei einer Temperatur von +37 ˚C und einer Wellenlänge von 405 nm vom automatischen Gerinnungsmessgerät aufgezeichnet und daraus die Extinktionsänderung als Maß für die F Xa-Aktivität in ΔOD/min bestimmt. Die Ergebnisse, die für die zwei Proben einer bestimmten F Xa-Flüssigzubereitung parallel bestimmt wurden, wurden gemittelt.

In den Tabellen 2 und 3 sind die stabilisierenden Effekte verschiedener Kombinationen von mehreren erfindungsgemäßen Stabilisatoren in einer F Xa-Flüssigzubereitung gezeigt. Zum Zeitpunkt t = 0 wurde ein chromogener F Xa-Aktivitätstest wie oben beschrieben durchgeführt und die F Xa-Aktivität anhand der Extinktionsänderung in ΔOD/min quantifiziert. 24 bzw. 48 Stunden nach Beginn der Inkubation bei +52 ˚C wurde die verbliebene F Xa-Aktivität jedes Testansatzes bestimmt und die prozentuale Abnahme gegenüber der Aktivität zum Zeitpunkt t = 0 ermittelt. Die Bildung des Quotienten aus der prozentualen Abnahme des Messsignals der Kontrollprobe ohne erfindungsgemäßen Stabilisator ("Kontrolle") und der prozentualen Abnahme des Messsignals einer erfindungsgemäßen Zubereitung ("Probe"), jeweils zum Zeitpunkt t = 24 h bzw. t = 48 h (Abnahme Aktivität Kontrolle / Abnahme Aktivität Probe) veranschaulicht den stabilisierenden Effekt einer erfindungsgemäßen Komposition. Quotienten > 1 deuten auf einen stabilisierenden Effekt der zugesetzten Substanz bzw. Substanzkombination hin; Quotienten < 1 würden auf einen destabilisierenden Effekt der zugesetzten Substanz bzw. Substanzkombination hindeuten; ein Quotient = 1 deutet darauf hin, dass eine zugesetzte Substanz bzw. Substanzkombination keinen Effekt auf die Stabilität hat.

Wie aus Tabelle 2 ersichtlich ist, wird die F Xa-Aktivität in Anwesenheit einer Kombination von Glycerin mit Natrium-Aspartat und/oder mit Natrium-Glutamat wesentlich besser stabilisiert als in Anwesenheit der einzelnen Additive. Die zusätzliche Zugabe von Ammoniumacetat verstärkt den stabilisierenden Effekt noch weiter.

Aus Tabelle 3 geht hervor, dass die F Xa-Aktivität in Anwesenheit von Ammoniumacetat und besonders in Anwesenheit von Ammoniumacetat zusammen mit Natrium-Aspartat und/oder Natrium-Glutamat oder zusammen mit Glycerin besser stabilisiert als in Abwesenheit von Ammoniumacetat.

Weitere Ergebnisse der Stabilitätsstudien sind in den Figuren 1-3 und den dazugehörigen Figurenbeschreibungen dargestellt.

**Tabelle 2: Stabilisierung von F Xa durch den Zusatz einer Kombination von Glycerin und einer oder mehrerer Aminosäuren**

| F Xa-Zubereitung | Signal [dOD/min] | | prozentuale Abnahme Signal [%] | Signal [dOD/min] Zeitpunkt 48 h | prozentuale Abnahme Signal [%] | Abnahme Aktivität Kontrolle / Abnahme Aktivität Probe |
|---|---|---|---|---|---|---|
| | Zeitpunkt 0h | Zeitpunkt 24 h | | | | |
| Kontrolle (F Xa in Puffer 1) | 1,413 | 0,885 | 37,4 | 0,703 | 50,2 | 1 |
| + 33 % Glycerin Vergleichsexperimente | 1,532 | 1,178 | 23,1 | 1,001 | 34,7 | 1,45 |
| + 200 mM Natriumglutamat Vergleichsexperimente | 1,624 | 1,355 | 16,4 | 1,157 | 28,6 | 1,76 |
| + 200 mM Natriumaspartat Vergleichsexperimente | 1,635 | 1,377 | 15,8 | 1,201 | 26,5 | 1,89 |
| + 33% Glycerin<br>+ 200 mM Natriumglutamat | 1,759 | 1,590 | 9,6 | 1,460 | 17,0 | 2,95 |
| + 33 % Glycerin<br>+ 200 mM Natriumaspartat | 1,865 | 1,682 | 9,8 | 1,554 | 16,7 | 3,00 |
| + 33 % Glycerin<br>+ 200 mM Natriumaspartat<br>+ 200 mM Natriumglutamat | 2,108 | 2,015 | 4,4 | 1,915 | 9,2 | 5,46 |
| + 33 % Glycerin<br>+ 200 mM Natriumglutamat<br>+ 300 mM Ammoniumacetat | 1,894 | 1,764 | 6,9 | 1,666 | 12,0 | 4,18 |

(fortgesetzt)

| F Xa-Zubereitung | Signal [dOD/min] | | prozentuale Abnahme Signal [%] | Signal [dOD/min] Zeitpunkt 48 h | prozentuale Abnahme Signal [%] | $\frac{\text{Abnahme Aktivität Kontrolle}}{\text{Abnahme Aktivität Probe}}$ |
|---|---|---|---|---|---|---|
| | Zeitpunkt 0h | Zeitpunkt 24 h | | | | |
| + 33 % Glycerin<br>+ 200 mM Natriumaspartat<br>+ 300 mM Ammoniumacetat | 1,883 | 1,779 | 5,5 | 1,689 | 10,3 | 4,87 |
| + 33 % Glycerin<br>+ 200 mM Natriumaspartat<br>+ 200 mM Natriumglutamat<br>+ 300 mM Ammoniumacetat | 2,069 | 1,975 | 4,5 | 1,880 | 9,1 | 5,52 |

**Tabelle 3: Stabilisierung von F Xa durch den Zusatz von Ammoniumacetat**

| F Xa-Zubereitung | Signal [dOD/min] | | prozentuale Abnahme Signal [%] | Signal [dOD/min] Zeitpunkt 48 h | prozentuale Abnahme Signal [%] | $\frac{\text{Abnahme Aktivität Kontrolle}}{\text{Abnahme Aktivität Probe}}$ |
|---|---|---|---|---|---|---|
| | Zeitpunkt 0 h | Zeitpunkt 24 h | | | | |
| Kontrolle (F Xa in Puffer 1) | 1,413 | 0,885 | 37,4 | 0,703 | 50,2 | 1 |
| + 300 mM Ammoniumacetat | 1,513 | 1,064 | 29,7 | 0,909 | 39,9 | 1,26 |
| + 300 mM Ammoniumacetat + 200 mM Natriumglutamat | 1,702 | 1,486 | 12,7 | 1,322 | 22,3 | 2,25 |
| + 300 mM Ammoniumacetat + 200 mM Natriumaspartat | 1,711 | 1,489 | 13,0 | 1,329 | 22,3 | 2,25 |
| + 300 mM Ammoniumacetat + 200 mM Natriumaspartat + 200 mM Natriumglutamat | 1,778 | 1,666 | 6,3 | 1,526 | 14,2 | 3,54 |
| + 300 mM Ammoniumacetat +33% Glycerin | 1,658 | 1,385 | 16,5 | 1,237 | 25,4 | 1,98 |

**Beispiel 2:**

**Herstellung von F Xa-Flüssigzubereitungen durch Zusatz von L-Lysin und deren Thermostabilität unter Stressbedingungen (Vergleichsexperiment)**

In Analogie zu den Experimenten in Beispiel 1 wurde ein Vergleichsexperiment durchgeführt, bei dem F Xa-Zubereitungen durch die Verwendung von Puffer 1 hergestellt wurden, der unterschiedliche Konzentrationen der Aminosäure L-Lysin enthielt. Die destabilisierende Wirkung von L-Lysin ist in Figur 3 und der dazugehörigen Figurenbeschreibung dargestellt.

**Beispiel 3:**

**Herstellung erfindungsgemäßer Thrombin-Flüssigzubereitungen durch Zusatz von Ammoniumacetat**

Es wurde ein käufliches Thrombin-Lyophilisat verwendet. Dabei handelte es sich um lyophilisiertes bovines Thrombin mit Zusatz von Heparin, Mannitol, NaCl und Aprotinin. Das Lyophilisat wurde in Puffer 2 gelöst (12 g/L Tris, 9 g/L NaCl, pH 8,2) oder in Puffer 2, der zusätzlich Ammoniumacetat in verschiedenen Konzentrationen enthielt. Die so erhaltenen Thrombin-Zubereitungen enthielten etwa 4 bis 5 IU Thrombin pro mL.

Zur Untersuchung der Stabilität der Thrombin-Zubereitungen unter beschleunigten Stressbedingungen wurden von jeder der erfindungsgemäßen Thrombin-Flüssigzubereitungen, die Ammoniumacetat enthielt, jeweils zwei Proben angesetzt. Alle erfindungsgemäßen Thrombin-Proben sowie die Thrombin-Kontrollproben, die Thrombin im gleichen Puffer ohne Zusatz von Ammoniumacetat enthielten, wurden für mindestens 48 Stunden bei +52 ˚C in einem Thermoblock inkubiert.

Zur Bestimmung der Thrombin-Aktivität in einer Probe wurden zum Zeitpunkt t = 0, d. h. unmittelbar nach dem Lösen des Thrombin-Lyophilisats in Puffer 2 und vor der Erwärmung auf +52 ˚C und dann nach verschiedenen Inkubationszeiten bei +52 ˚C jeweils 175 μL-Aliquots entnommen, und die Thrombin-Aktivität wurde auf dem automatischen Gerinnungsmessgerät Sysmex® CA-7000 (Sysmex Corporation, Kobe, Japan) bestimmt. Dazu wurde das jeweilige Aliquot vom Gerinnungsautomaten zunächst mit 24 μL Reaktionspuffer gemischt. Nach einer Inkubation für 180 Sekunden bei +37 ˚C wurden jeder Probe 33 μl Substrat-Reagenz (Tos-Gly-Pro-Arg-ANBA-isopropylamid, 2 mM) zugegeben. Die Thrombin-abhängige Bildung des Chromophors ANBA (5-Amino-2-Nitro-Benzoesäure) wurde bei einer Temperatur von +37 ˚C und einer Wellenlänge von 405 nm von dem automatischen Gerinnungsmessgerät aufgezeichnet und die Extinktionsänderung als Maß für die Thrombin-Aktivität in ΔOD/min bestimmt. Die Ergebnisse, die für die zwei Proben einer bestimmten Thrombin-Flüssigzubereitung parallel bestimmt wurden, wurden gemittelt.

Die Ergebnisse der Stabilitätsstudie sind in Figur 4 und der dazugehörigen Figurenbeschreibung dargestellt.

## Patentansprüche

1. Zubereitung, die mindestens eine Serin-Endopeptidase enthält, **dadurch gekennzeichnet, dass**

    a) Ammoniumacetat und/oder
    b) mindestens eine Polyaminosäure und/oder
    c) Glycerin zusammen mit mindestens einer Aminosäure aus der Gruppe Asparaginsäure und deren Salze, Glutaminsäure und deren Salze, Histidin und Glycin

    enthalten ist.

2. Zubereitung gemäß Anspruch 1, welche

    a) Ammoniumacetat und/oder
    b) mindestens eine Polyaminosäure enthält,

    **dadurch gekennzeichnet, dass** zusätzlich Glycerin enthalten ist.

3. Zubereitung gemäß Anspruch 1, welche

    a) Ammoniumacetat und/oder
    b) mindestens eine Polyaminosäure enthält,

**dadurch gekennzeichnet, dass** zusätzlich mindestens eine Aminosäure aus der Gruppe Asparaginsäure und deren Salze, Glutaminsäure und deren Salze, Histidin und Glycin enthalten ist.

4. Zubereitung nach einem der Ansprüche 1 bis 3 **dadurch gekennzeichnet, dass** Ammoniumacetat in einer Endkonzentration von 25 bis 1000 mM, bevorzugt von 400 bis 1000 mM, besonders bevorzugt von 700 bis 1000 mM enthalten ist.

5. Zubereitung nach einem der Ansprüche 1 bis 4 **dadurch gekennzeichnet, dass** mindestens eine Polyaminosäure aus der Gruppe Poly-L-Glutamat und Poly-L-Aspartat enthalten ist.

6. Zubereitung nach einem der Ansprüche 1 bis 5 **dadurch gekennzeichnet, dass** eine Polyaminosäure in einer Endkonzentration von 1 bis 10 mM, bevorzugt von 2 bis 10 mM, besonders bevorzugt von 2 bis 5 mM enthalten ist.

7. Zubereitung nach einem der Ansprüche 1 bis 6 **dadurch gekennzeichnet, dass** Glycerin in einer Endkonzentration von 0,5 bis 50 Volumenprozent, bevorzugt von 10 bis 50 Volumenprozent, besonders bevorzugt von 30 bis 50 Volumenprozent enthalten ist.

8. Zubereitung nach einem der Ansprüche 1 bis 7 **dadurch gekennzeichnet, dass** mindestens eine Aminosäure aus der Gruppe Histidin und Glycin in einer Endkonzentration von 10 bis 250 mM, bevorzugt von 25 bis 200 mM, besonders bevorzugt von 100 bis 150 mM enthalten ist.

9. Zubereitung gemäß einem der Ansprüche 1 bis 8 **dadurch gekennzeichnet, dass** mindestens eine Aminosäure aus der Gruppe, Asparaginsäure und deren Salze und Glutaminsäure und deren Salze in einer Endkonzentration von 10 bis 1000 mM, bevorzugt von 400 bis 1000 mM, besonders bevorzugt von 500 bis 800 mM enthalten ist.

10. Zubereitung nach einem der Ansprüche 1 bis 9 **dadurch gekennzeichnet, dass** weiterhin mindestens ein nichtreduzierender Zucker enthalten ist.

11. Zubereitung nach einem der Ansprüche 1 bis 10 **dadurch gekennzeichnet, dass** die enthaltene Serin-Endopeptidase ein tierischer oder humaner Blutgerinnungsfaktor aus der Gruppe F II, F VII, F IX, F X, F XI, F XII, F IIa, F VIIa, F IXa, F Xa, F XIa und F XIIa ist.

12. Zubereitung nach einem der Ansprüche 1 bis 10 **dadurch gekennzeichnet, dass** die enthaltene Serin-Endopeptidase ein tierischer oder humaner Komplementfaktor aus der Gruppe C1r, C1s, Komplementfaktor D und Komplementfaktor I ist.

13. Zubereitung nach einem der Ansprüche 1 bis 10 **dadurch gekennzeichnet, dass** die enthaltene Serin-Endopeptidase aus folgender Gruppe stammt: Chymotrypsin, Trypsin, Plasmin, Acrosin, Cathepsin G, Plasma-Kallikrein, Gewebs-Kallikrein, Pankreas-Elastase, Leukozyten-Elastase, C3/C5 Konvertase (klassisch), C3/C5 Konvertase (alternativ), Subtilisin, Proteinase K, aktiviertes Protein C, Gewebs-Plasminogen-Aktivator, Urokinase-Plasminogen-Aktivator, Furin, Limulus Gerinnungsfaktor C, Limulus Gerinnungsfaktor B, Limulus Gerinnungsenzym und Schlangengift Faktor V Aktivator.

14. Zubereitung nach einem der Ansprüche 1 bis 13 **dadurch gekennzeichnet, dass** die Zubereitung flüssig ist.

15. Verwendung einer Zubereitung gemäß Anspruch 11 in einem Testverfahren zur Bestimmung eines Gerinnungsparameters.

16. Verwendung einer Zubereitung gemäß Anspruch 11, welche F Xa oder F IIa enthält, in einem Testverfahren zur Bestimmung von Antithrombin.

17. Verwendung einer Zubereitung gemäß Anspruch 11, welche F Xa enthält, in einem Testverfahren zur Bestimmung von Heparin.

## Claims

1. Preparation which contains at least one serine endopeptidase, **characterized in that**

a) ammonium acetate and/or
b) at least one polyamino acid and/or
c) glycerol together with at least one amino acid from the group aspartic acid and its salts, glutamic acid and its salts, histidine and glycine

is present.

**2.** Preparation according to claim 1, which contains

a) ammonium acetate and/or
b) at least one polyamino acid,

**characterized in that** glycerol is additionally present.

**3.** Preparation according to claim 1, which contains

a) ammonium acetate and/or
b) at least one polyamino acid,

**characterized in that** additionally at least one amino acid from the group aspartic acid and its salts, glutamic acid and its salts, histidine and glycine is present.

**4.** Preparation according to one of claims 1 to 3, **characterized in that** ammonium acetate is present in a final concentration of 25 to 1000 mM, preferably of 400 to 1000 mM, particularly preferably of 700 to 1000 mM.

**5.** Preparation according to one of claims 1 to 4, **characterized in that** at least one polyamino acid from the group poly-L-glutamate and poly-L-aspartate is present.

**6.** Preparation according to one of claims 1 to 5, **characterized in that** a polyamino acid is present in a final concentration of 1 to 10 mM, preferably of 2 to 10 mM, particularly preferably of 2 to 5 mM.

**7.** Preparation according to one of claims 1 to 6, **characterized in that** glycerol is present in a final concentration of 0.5 to 50 percent by volume, preferably of 10 to 50 percent by volume, particularly preferably of 30 to 50 percent by volume.

**8.** Preparation according to one of claims 1 to 7, **characterized in that** at least one amino acid from the group histidine and glycine is present in a final concentration of 10 to 250 mM, preferably of 25 to 200 mM, particularly preferably of 100 to 150 mM.

**9.** Preparation according to one of claims 1 to 8, **characterized in that** at least one amino acid from the group aspartic acid and its salts and glutamic acid and its salts is present in a final concentration of 10 to 1000 mM, preferably of 400 to 1000 mM, particularly preferably of 500 to 800 mM.

**10.** Preparation according to one of claims 1 to 9, **characterized in that** furthermore at least one nonreducing sugar is present.

**11.** Preparation according to one of claims 1 to 10, **characterized in that** the serine endopeptidase present is an animal or human blood clotting factor from the group F II, F VII, F IX, F X, F XI, F XII, F IIa, F VIIa, F IXa, F Xa, F XIa and F XIIa.

**12.** Preparation according to one of claims 1 to 10, **characterized in that** the serine endopeptidase present is an animal or human complement factor from the group C1r, C1s, complement factor D and complement factor I.

**13.** Preparation according to one of claims 1 to 10, **characterized in that** the serine endopeptidase present originates from the following group: chymotrypsin, trypsin, plasmin, acrosin, cathepsin G, plasma kallikrein, tissue kallikrein, pancreas elastase, leucocyte elastase, C3/C5 convertase (classical), C3/C5 convertase (alternative), subtilisin, proteinase K, activated protein C, tissue plasminogen activator, urokinase plasminogen activator, furin, limulus clotting factor C, limulus clotting factor B, limulus clotting enzyme and snake venom factor V activator.

**14.** Preparation according to one of claims 1 to 13, **characterized in that** the preparation is liquid.

**15.** Use of a preparation according to claim 11 in a test procedure for the determination of a clotting parameter.

**16.** Use of a preparation according to claim 11, which contains F Xa or F IIa, in a test procedure for the determination of antithrombin.

**17.** Use of a preparation according to claim 11, which contains F Xa, in a test procedure for the determination of heparin.

**Revendications**

**1.** Préparation contenant au moins une sérine endopeptidase, **caractérisée en ce qu'**elle contient :

a) de l'acétate d'ammonium, et/ou
b) au moins un polyaminoacide, et/ou
c) du glycérol, conjointement avec au moins un acide aminé choisi dans le groupe constitué de l'acide aspartique et de ses sels, de l'acide glutamique et de ses sels, de l'histidine et de la glycine.

**2.** Préparation selon la revendication 1, qui contient :

a) de l'acétate d'ammonium, et/ou
b) au moins un polyaminoacide,

**caractérisée en ce qu'**elle contient, en outre, du glycérol.

**3.** Préparation selon la revendication 1, qui contient :

a) de l'acétate d'ammonium, et/ou
b) au moins un polyaminoacide,

**caractérisée en ce qu'**elle contient, en outre, au moins un acide aminé choisi dans le groupe constitué de l'acide aspartique et de ses sels, de l'acide glutamique et de ses sels, de l'histidine et de la glycine.

**4.** Préparation selon l'une quelconque des revendications 1 à 3, **caractérisée en ce qu'**elle contient de l'acétate d'ammonium à une concentration finale de 25 à 1000 mM, de préférence, de 400 à 1000 mM, mieux encore, de 700 à 1000 mM.

**5.** Préparation selon l'une quelconque des revendications 1 à 4, **caractérisée en ce qu'**elle contient au moins un polyaminoacide choisi dans le groupe comprenant le poly-L-glutamate et le poly-L-aspartate.

**6.** Préparation selon l'une quelconque des revendications 1 à 5, **caractérisée en ce qu'**elle contient un polyaminoacide à une concentration finale de 1 à 10 mM, de préférence, de 2 à 10 mM, mieux encore, de 2 à 5 mM.

**7.** Préparation selon l'une quelconque des revendications 1 à 6, **caractérisée en ce qu'**elle contient du glycérol à une concentration finale de 0,5 à 50 pour-cent en volume, de préférence, de 10 à 50 pour-cent en volume, mieux encore, de 30 à 50 pour-cent en volume.

**8.** Préparation selon l'une quelconque des revendications 1 à 7, **caractérisée en ce qu'**elle contient au moins un acide aminé choisi dans le groupe constitué de l'histidine et de la glycine à une concentration finale de 10 à 250 mM, de préférence, de 25 à 200 mM, mieux encore, de 100 à 150 mM.

**9.** Préparation selon l'une quelconque des revendications 1 à 8, **caractérisée en ce qu'**elle contient au moins un acide aminé choisi dans le groupe constitué de l'acide aspartique et de ses sels et de l'acide glutamique et de ses sels, à une concentration finale de 10 à 1000 mM, de préférence, de 400 à 1000 mM, mieux encore, de 500 à 800 mM.

**10.** Préparation selon l'une quelconque des revendications 1 à 9, **caractérisée en ce qu'**elle contient, en outre, au moins un sucre non réducteur.

**11.** Préparation selon l'une quelconque des revendications 1 à 10, **caractérisée en ce que** la sérine endopeptidase contenue est un facteur de coagulation sanguine d'origine animale ou humaine, choisi parmi les facteurs F II, F VII, F IX, F X, F XI, F XII, F IIa, F VIIa, F IXa, F Xa, F XIa et F XIIa.

**12.** Préparation selon l'une quelconque des revendications 1 à 10, **caractérisée en ce que** la sérine endopeptidase contenue est un facteur du complément d'origine animale ou humaine, choisi dans le groupe constitué du C1r, du C1s, du facteur du complément D et du facteur du complément I.

**13.** Préparation selon l'une quelconque des revendications 1 à 10, **caractérisée en ce que** la sérine endopeptidase contenue provient du groupe comprenant : la chymotrypsine, la trypsine, la plasmine, l'acrosine, la cathépsine G, la kallikréine plasmatique, la kallikréine tissulaire, l'élastase pancréatique, l'élastase leucocytaire, la C3/C5 convertase (classique), la C3/C5 convertase (variante), la subtilisine, la protéinase K, la protéine C activée, l'activateur tissulaire du plasminogène, l'activateur du plasminogène de type urokinase, la furine, le facteur C de coagulation de la limule, le facteur B de coagulation de la limule, l'enzyme de coagulation de la limule et l'activateur du facteur V de venin de serpent.

**14.** Préparation selon l'une quelconque des revendications 1 à 13, **caractérisée en ce que** la préparation est liquide.

**15.** Utilisation d'une préparation selon la revendication 11 dans un procédé d'essai pour déterminer un paramètre de coagulation.

**16.** Utilisation d'une préparation selon la revendication 11, qui contient le facteur F Xa ou F IIa, dans un procédé d'essai pour déterminer l'antithrombine.

**17.** Utilisation d'une préparation selon la revendication 11, qui contient le facteur F Xa, dans un procédé d'essai pour déterminer l'héparine.

Figur 1

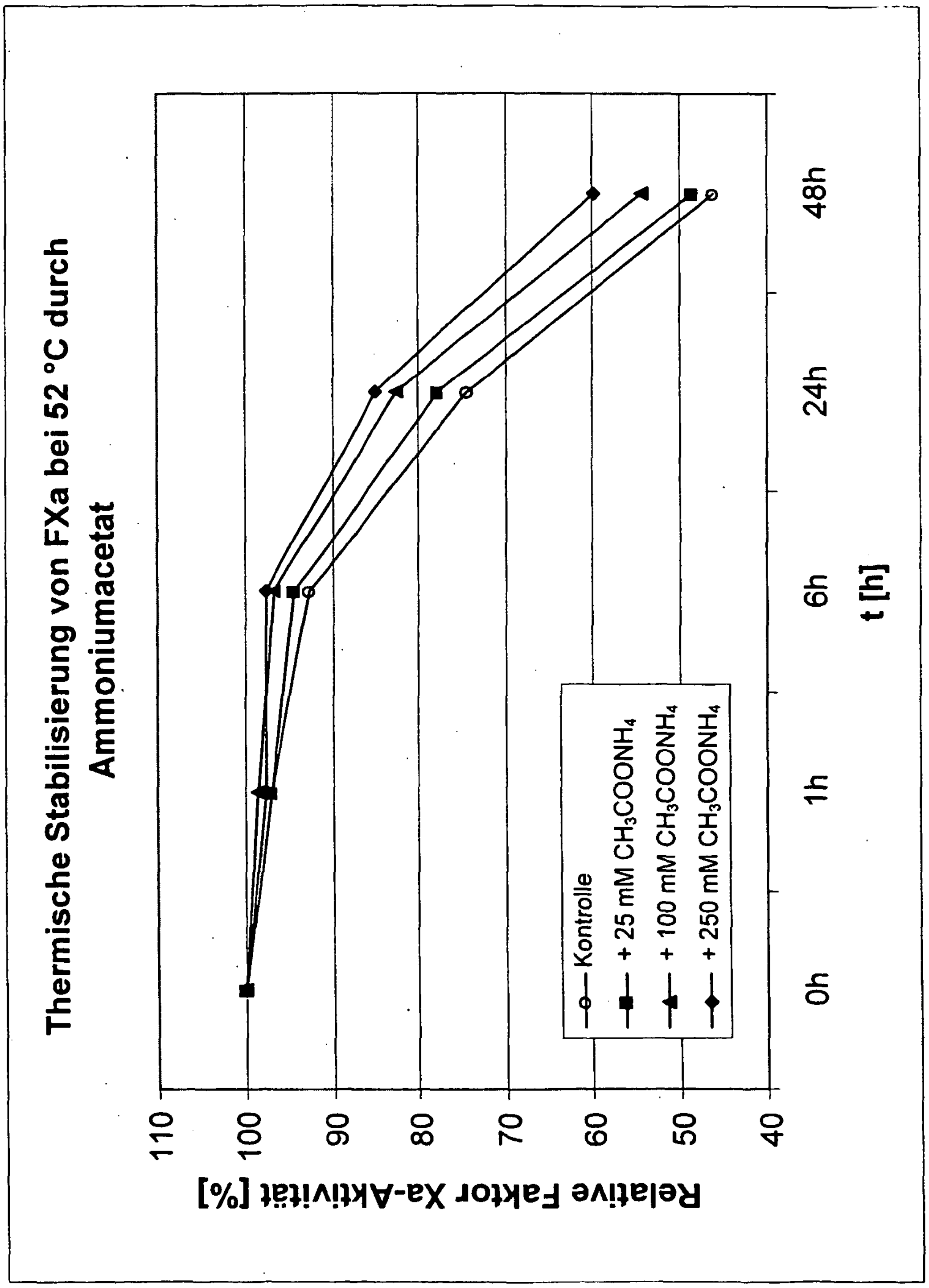
Thermische Stabilisierung von FXa bei 52 °C durch Ammoniumacetat
Relative Faktor Xa-Aktivität [%]
110
100
90
80
70
60
50
40
0h
1h
6h
24h
48h
t [h]
Kontrolle
+ 25 mM CH3COONH4
+ 100 mM CH3COONH4
+ 250 mM CH3COONH4

Figur 2

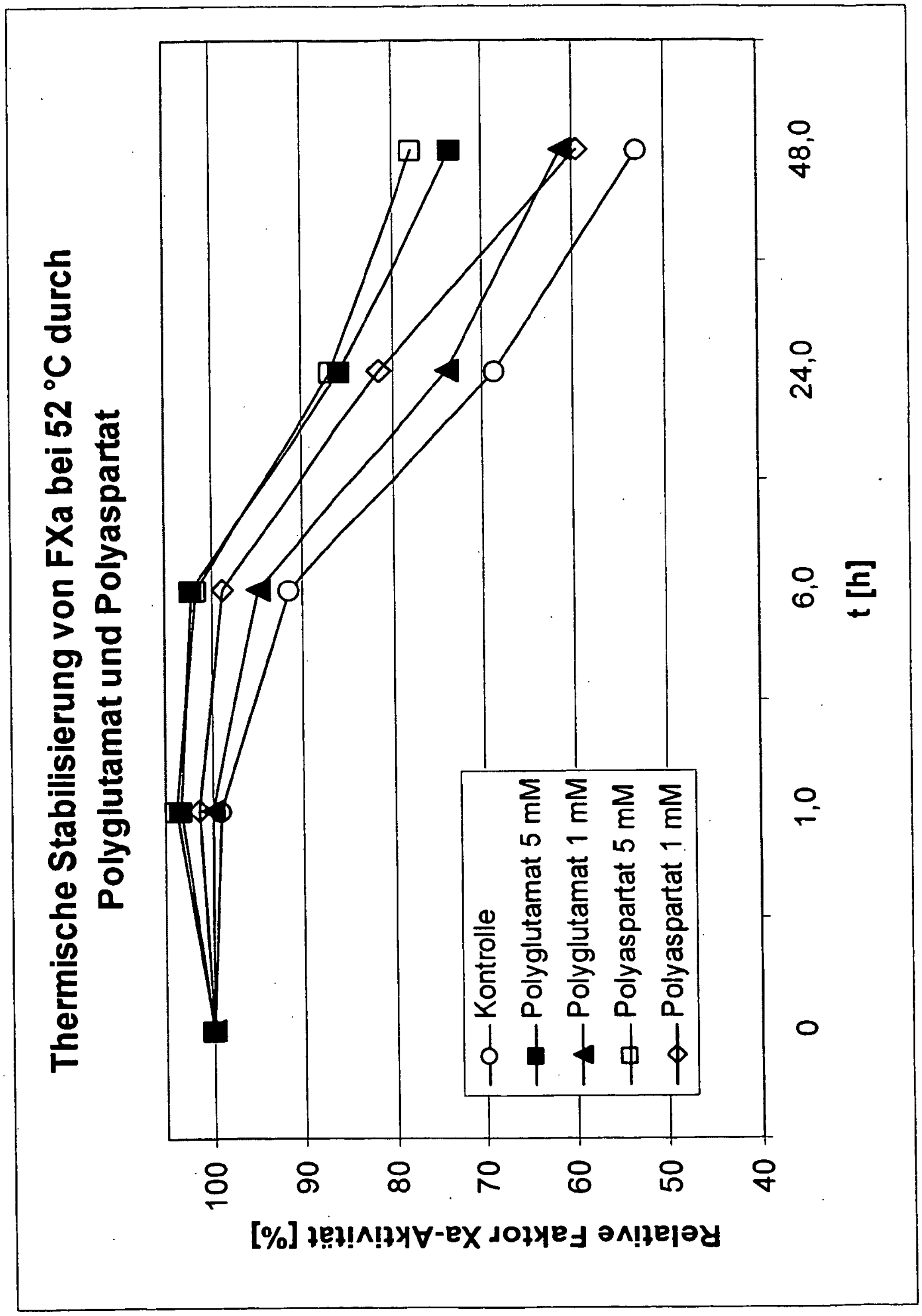
Thermische Stabilisierung von FXa bei 52 °C durch Polyglutamat und Polyaspartat
Relative Faktor Xa-Aktivität [%]
100
90
80
70
60
50
40
0
1,0
6,0
24,0
48,0
t [h]
Kontrolle
Polyglutamat 5 mM
Polyglutamat 1 mM
Polyaspartat 5 mM
Polyaspartat 1 mM

Figur 3

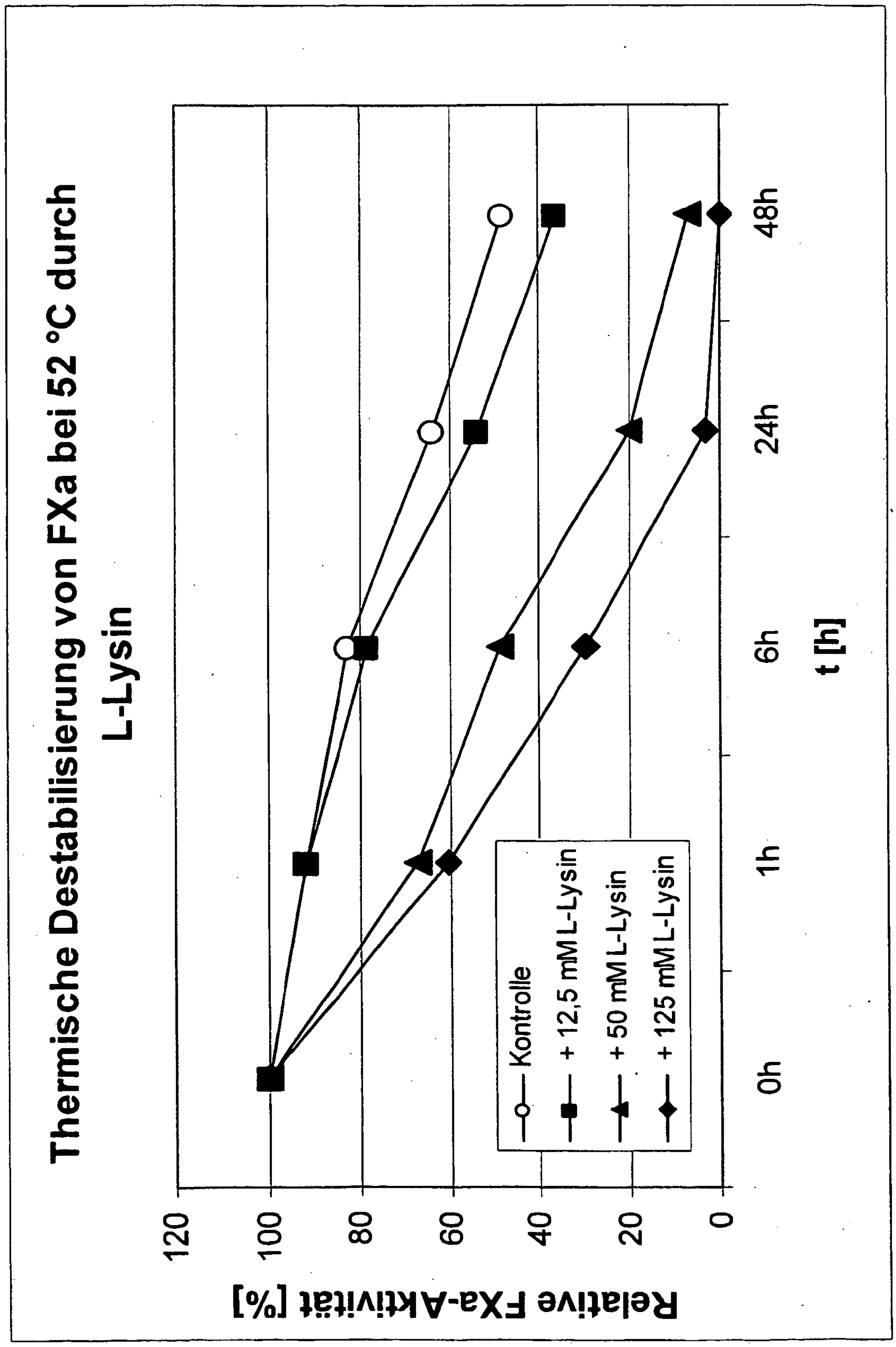
Thermische Destabilisierung von FXa bei 52 °C durch L-Lysin
Relative FXa-Aktivität [%]
120
100
80
60
40
20
0
0h
1h
6h
24h
48h
t [h]
Kontrolle
+ 12,5 mM L-Lysin
+ 50 mM L-Lysin
+ 125 mM L-Lysin

Figur 4

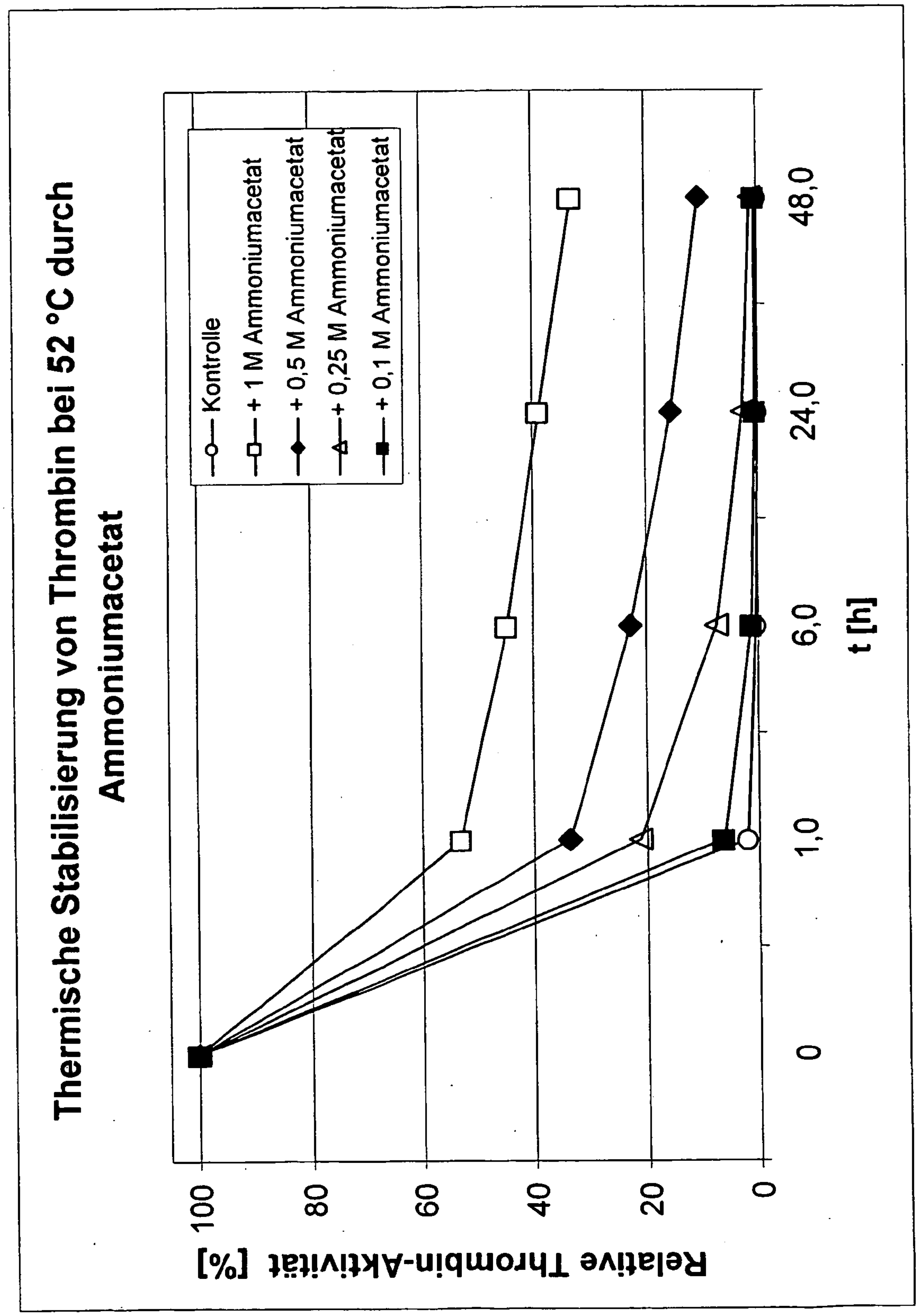
Thermische Stabilisierung von Thrombin bei 52 °C durch Ammoniumacetat
Relative Thrombin-Aktivität [%]
100
80
60
40
20
0
0
1,0
6,0
24,0
48,0
t [h]
Kontrolle
+ 1 M Ammoniumacetat
+ 0,5 M Ammoniumacetat
+ 0,25 M Ammoniumacetat
+ 0,1 M Ammoniumacetat

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6514940 B2
- DE 4325872 C1
- EP 680764 A2
- EP 1153608 A1
- EP 216179 B1
- US 5308755 A


### In der Beschreibung aufgeführte Nicht-Patentliteratur


- **BAJAJ, S. P. ; MANN, K. G.** Simultaneous purification of bovine prothrombin and Factor X. *J. Biol. Chem.,* 1973, vol. 248, 7729-7741